# EUROPÄISCHE PATENTSCHRIFT

(11) **EP 3 145 511 B1**
(45) Veröffentlichungstag und Bekanntmachung des Hinweises auf die Patenterteilung: **05.01.2022**
(21) Anmeldenummer: 15729760.7
(22) Anmeldetag: 22.05.2015
(51) Int. Cl.: A61K 31/505, A61K 9/00, A61P 27/02, A61P 27/04

(54) **ZUSAMMENSETZUNG ZUR BEHANDLUNG DES AUGES**
COMPOSITION FOR TREATING THE EYE
COMPOSITION POUR LE TRAITEMENT DE L' OEIL

(30) Priorität: 22.05.2014 DE 102014007423
(43) Veröffentlichungstag der Anmeldung: 29.03.2017
(60) Teilanmeldung: 21209678.8
(73) Patentinhaber: bitop AG, 58453 Witten (DE)
(72) Erfinder: BILSTEIN, Andreas, 50129 Bergheim (DE); GALLA, Hans-Joachim, 48161 Münster (DE); DWIVEDI, Mridula, 44227 Dortmund (DE)
(74) Vertreter: Heide, Anna Katharina
(86) Internationale Anmeldenummer: PCT/EP2015/061434
(87) Internationale Veröffentlichungsnummer: WO 2015/177353

(56) Entgegenhaltungen:
- CN-A- 101 491 525
- Anonymous: "Hylo-Protect", , 1. Juli 2013 (2013-07-01), XP002742588, Gefunden im Internet: URL:https://www.mediherz-shop.de/images/pr oducts/mediherz/beipackzettel/09715226_d.p df
- MRIDULA DWIVEDI ET AL: "Biophysical investigations of the structure and function of the tear fluid lipid layer and the effect of ectoine. Part A: Natural meibomian lipid films", BIOCHIMICA ET BIOPHYSICA ACTA (BBA) - BIOMEMBRANES, Bd. 1838, Nr. 10, 17. Mai 2014 (2014-05-17), Seiten 2708-2715, XP055203902, ISSN: 0005-2736, DOI: 10.1016/j.bbamem.2014.05.011
- MRIDULA DWIVEDI ET AL: "Biophysical investigations of the structure and function of the tear fluid lipid layers and the effect of ectoine. Part B: Artificial lipid films", BIOCHIMICA ET BIOPHYSICA ACTA (BBA) - BIOMEMBRANES, Bd. 1838, Nr. 10, 20. Mai 2014 (2014-05-20), Seiten 2716-2727, XP055203901, ISSN: 0005-2736, DOI: 10.1016/j.bbamem.2014.05.007
- None

## Beschreibung

Die Erfindung betrifft eine Zusammensetzung, die der Behandlung von Erkrankungen des Auges dient, die mit einer Störung des Tränenfilms in Zusammenhang stehen, insbesondere des Syndroms des trockenen Auges (Keratoconjunctivitis sicca).

Die Tränendrüsen (Glandulae lacrimales) des Menschen sind für die Produktion der Tränenflüssigkeit verantwortlich. Es handelt sich um eine klare, leicht alkalische Flüssigkeit, die der Befeuchtung der Bindehaut und der Hornhaut dient. Störungen bei der Tränenproduktion oder in der Zusammensetzung des Tränenfilms können zum Syndrom des trockenen Auges (Keratoconjunctivitis sicca, Keratitis sicca; engl. Drye Eye Syndrome, DES) führen. Hiermit verbundene Symptome sind ein Fremdkörpergefühl, Brennen und Augenrötung. In schweren Fällen kann es zu einer Hornhautschädigung bis hin zur Erblindung kommen. Die Keratoconjunctivitis sicca ist eine häufig vorkommende Erkrankung, die ca. 10 bis 20 % der erwachsenen Bevölkerung betrifft. Die Behandlung erfolgt häufig mit Hyaluronsäure, künstlicher Tränenflüssigkeit oder Cellulosederivaten. Sie ist jedoch häufig unbefriedigend aufgrund unzureichenden Behandlungserfolgs oder Nebenwirkungen.

Neben den Tränendrüsen sind die Meibom-Drüsen (Glandulae tarsales) von Bedeutung. Hierbei handelt es sich um Talgdrüsen am Augenlidrand. Die Meibom-Drüsen geben eine ölige Flüssigkeit ab, die sich mit der Tränenflüssigkeit vermischt. Insgesamt ergibt sich eine äußere Lipidschicht auf dem Tränenfilm, die bewirkt, dass die wässrige Tränenflüssigkeit nicht zu schnell verdunstet. Störungen in der Sekretion der Meibom-Drüsen, beispielsweise in der Lipidzusammensetzung des Sekrets, oder ein Reißen der Lipidschicht, führen somit zu vorzeitiger Verdunstung der Tränenflüssigkeit und begünstigen damit die Entstehung einer Keratoconjunctivitis sicca.

Es stellt sich somit die Aufgabe, eine Zusammensetzung zur Verfügung zu stellen, die ein Reißen der Lipidschicht, verbunden mit erhöhter Verdunstung von Tränenflüssigkeit und damit das Auftreten von Augenerkrankungen, die hiermit in Zusammenhang stehen, verhindert. Insbesondere soll die Zusammensetzung der Entstehung einer Keratoconjunctivitis sicca vorbeugen bzw. diese behandeln.

Diese Aufgabe wird erfindungsgemäß gelöst durch eine Zusammensetzung enthaltend als Wirkstoff Ectoin, Hydroxyectoin und/oder Salze, Ester oder Amide dieser Verbindungen zur Verwendung in einem Verfahren zur Behandlung und/oder Prophylaxe von Erkrankungen des Auges, die mit einer Störung des Tränenfilms in Zusammenhang stehen, wobei die Erkrankung eine hyperevaporative Keratoconjunctivitis sicca ist, die auf eine Fehlfunktion der Meibom-Drüsen zurückzuführen ist, und der Wirkstoff eine Fluidisierung der durch die Meibom-Drüsen erzeugten Lipidschicht bewirkt und wobei die Zusammensetzung in Form von Liposomen vorliegt.

Bei Ectoin und Hydroxyectoin handelt es sich um Tetrahydropyrimidinderivate, die unter Stressbedingungen von extremophilen, insbesondere halophilen Mikroorganismen synthetisiert werden. Für Ectoin und Hydroxyectoin wurden bislang verschiedene Verwendungen beschrieben, beispielsweise als Moisturizer, zur Behandlung des Vascular Leak Syndroms (VLS) (DE 10 2006 056 766 A1) oder zur Behandlung von Neurodermitis (DE 103 30 243 A1). Aus der DE 100 06 578 A1 ist die Verwendung von Ectoin und seinen Derivaten zum Schutz von Biopolymeren vor dem Abbau durch degradierende Enzyme wie Proteasen, Nucleasen oder Lipasen bekannt.

Die systematische Bezeichnung für Ectoin lautet 2-Methyl-1,4,5,6-tetrahydropyrimidin-4-carbonsäure, für Hydroxyectoin 5-Hydroxy-2-methyl-1,4,5,6-tetrahydropyrimidin-4-carbonsäure.

Die Struktur des natürlichen L-Ectoins ((S)-2-Methyl-1,4,5,6-tetrahydropyrimidin-4-carbonsäure) ist im Folgenden dargestellt:

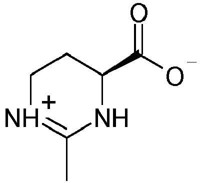

Die Struktur des natürlichen Hydroxyectoins ((4S,5S)-5-Hydroxy-2-methyl-1,4,5,6-tetrahydropyrimidin-4-carbonsäure) wird im Folgenden wiedergegeben:

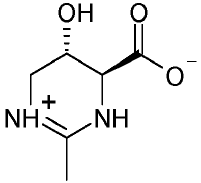

Die Verwendung der angegebenen Stereoisomere ist bevorzugt, jedoch nicht obligatorisch, d.h. auch die Verwendung anderer Stereoisomere bzw. des Racemats ist möglich.

Eine Zusammensetzung zur Befeuchtung trockener Augen, welche u. a. Ectoin enthält, wird unter der Bezeichnung HYLO^{®}-PROTECT von der Firma Ursapharm angeboten, das Produkt dient jedoch nicht speziell der Behebung von Problemen, die auf eine Fehlfunktion der Meibom-Drüsen zurückzuführen sind. Des Weiteren liegt die Zusammensetzung nicht in Form von Liposomen vor.

Der der Befeuchtung und Schutz von Bindehaut und Hornhaut dienende Tränenfilm besteht im Wesentlichen aus einer inneren mucinreichen Schicht, einer wässrigen Phase, die Proteine, Metaboliten und Salze enthält, sowie der äußeren Lipidschicht an der Grenzfläche zwischen Flüssigkeit und Luft. Die Lipidschicht setzt sich aus verschiedenen Lipiden zusammen, insbesondere polaren Phospholipiden und Fettsäuren, Cholesterinestern und Triacylglyceriden. Dabei bilden die polaren Lipide den untersten Teil der Lipidschicht, d. h. sie formen die Grenze zur darunterliegenden wässrigen Phase. Cholesterin- und andere Sterolester lagern sich zwischen den unpolaren Kopfgruppen der polaren Lipide ein, so dass sich eine Art Plattform für die weiter außen befindlichen unpolaren Anteile der Lipidschicht ausbildet, insbesondere die Triacylglyceride.

Die Erfindung beruht auf der Erkenntnis, dass einer der Gründe für ein vorzeitiges Reißen der Lipidschicht in ihrer erhöhten Starrheit bei Keratoconjunctivitis sicca-Patienten begründet ist. Bei den betroffenen Personen ist das Verhältnis von neutralen zu polaren Lipiden erhöht. Entsprechend konnte eine erhöhte Lipid-Lipid-Wechselwirkung im Vergleich zu Gesunden festgestellt werden. Die damit verbundene erhöhte Starrheit der Lipidschicht kann dazu führen, dass sich in dieser Schicht Lücken ausbilden, durch die die Tränenflüssigkeit mit der Luft in Kontakt kommt und in zu starkem Maße verdunstet.

Die erfindungsgemäße Zusammensetzung ist zur Behandlung der hyperevaporativen Keratoconjunctivitis sicca geeignet. Die Zusammensetzung eignet sich zur Behandlung einer Xerophthalmie, bei der man unterscheidet zwischen der hyperevaporativen Form, die durch eine Fehlfunktion der Meibom-Drüsen gekennzeichnet ist, und der hypovolämen Form, welche sich durch mangelnde Tränensekretion auszeichnet. Die Zusammensetzung ist geeignet zur Behandlung des Syndroms des trockenen Auges in seiner hyperevaporativen Form.

Es hat sich nun herausgestellt, dass Ectoin und Hydroxyectoin in der Lage sind, für eine Fluidisierung der Lipidschicht zu sorgen und damit einer Keratoconjunctivitis sicca vorzubeugen bzw. diese zu behandeln. Die erfindungsgemäße Zusammensetzung ist somit in der Lage, Störungen, die im Zusammenhang mit der durch die Meibom-Drüsen erzeugten Lipidschicht stehen, beispielsweise hinsichtlich der Zusammensetzung, zu beheben. Im Einzelnen vergrößert sich die Fläche, die die einzelnen Phospholipide einnehmen. Dies bewirkt eine verringerte Ordnung und größere Lücken in der Phospholipidschicht und damit weniger Plattform für die hydrophoben Bestandteile der Lipidschicht, insbesondere die Triacylglyceride. Da diese weniger Raum zur Verfügung haben, können sich auf der äußeren Lipidschicht kleine tröpfchenartige Strukturen aus Triacylglyceriden ausbilden. Insgesamt wird die Lipidschicht beweglicher und weniger starr; die Wahrscheinlichkeit des Reißens der Lipidschicht wird verringert. Darüber hinaus wird das Spreiten der Lipidschicht erleichtert, die Elastizität der Lipidschicht wird erhöht: Insbesondere bei Druckausübung können sich einige Lipide, vor allem die unpolaren

Triacylglyceride, zu tröpfchenartigen Strukturen zusammenfinden, bei Expansion können sich diese erneut ausbreiten, um eine stabile Lipidschicht zu gewährleisten.

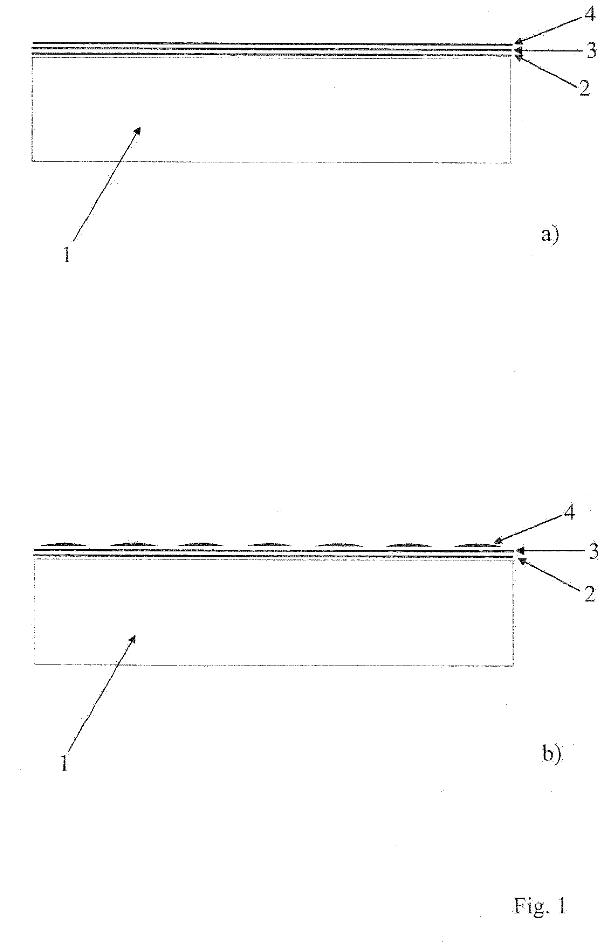

Ein makroskopisches Modell für den Effekt, den eine ausreichend hohe Ectoinkonzentration auf den Tränenfilm ausübt, ist in Figur 1 schematisch wiedergegeben. Unter a) wird der Tränenfilm ohne Zusatz von Ectoin gezeigt, wobei 1 die wässrige Phase zeigt. Die Grenzschicht 2 wird insbesondere durch polare Phospholipide gebildet, wobei teilweise die Cholesterinestermoleküle aus der darüber liegenden Schicht 3 interkalieren. Insgesamt bildet sich so eine hydrophobe Plattform für die unpolaren Triacylglyceride 4, die die äußere Grenzschicht zur Umgebung bilden.

Die Darstellung b) zeigt die Situation in Gegenwart von 100 mM Ectoin in der Tränenflüssigkeit. Die Moleküle in den an die wässrige Phase angrenzenden Schichten 2 und 3 der Lipidschicht sind deutlich weniger kompakt angeordnet als gemäß Darstellung a); sie bilden daher keine durchgehende Plattform mehr aus für die Triacylglyceride 4. Diese formen daher kleine tröpfchenartige lokale Ansammlungen an der Grenzschicht zur Luft. Insgesamt ist die Lipidschicht in Gegenwart von Ectoin deutlich beweglicher und weniger starr, was die Gefahr des Reißens herabsetzt. Darüber hinaus weist die Lipidschicht an den Stellen der tröpfchenartigen Ansammlungen eine Dicke von ca. 200 nm auf, während die Lipidschicht in Abwesenheit von Ectoin lediglich eine Dicke von ca. 20 nm hat.

Die erfindungsgemäße Zusammensetzung ist insbesondere für die lokale bzw. topische Anwendung vorgesehen. Entsprechend kann die Zusammensetzung in flüssiger Form vorliegen, beispielsweise in Form von Augentropfen. In der Regel handelt es sich um eine wässrige Lösung. Die Zusammensetzung kann bspw. isotonisch, hypotonisch oder hypertonisch sein. Daneben sind aber auch andere Darreichungsformen denkbar, beispielsweise Cremes oder Gele.

Als pharmakologisch verträgliche Salze des Ectoins/Hydroxyectoins kommen die Alkali- oder Erdalkalisalze, insbesondere die Salze des Kaliums, Natriums, Magnesiums und Calciums, aber auch Salze mit organischen Basen wie z. B. mit nicht toxischen aliphatischen oder aromatischen Aminen in Frage.

Durch Umsetzung der Carboxylgruppe des Ectoins/Hydroxyectoins mit Alkoholen oder Aminen, können entsprechende Ester oder Amide erhalten werden, die ebenfalls erfindungsgemäß einsetzbar sind. Im Falle eines Amids kann die Amidfunktion wiederum gesättigte oder ungesättigte, geradkettige oder verzweigte Alkylgruppen aufweisen. Beim Hydroxyectoin kann auch die Hydroxygruppe mit einer Carbonsäure unterschiedlicher Kettenlänge zu einem entsprechenden Ester umgesetzt sein.

Die Zusammensetzung kann übliche Hilfsstoffe enthalten, z. B. Trägermittel, Konservierungsmittel, Bakterizide, Lösungsvermittler, Vitamine, Stabilisatoren, Substanzen zum Verhindern des Schäumens, osmotisch aktive Substanzen, Farbstoffe, oberflächenaktive Substanzen, Emulgatoren, feuchthaltende Substanzen u.ä..

Konservierungsmittel sind beispielsweise Thiomersal, organische Quecksilberverbindungen wie Phenylquecksilber, Benzalkoniumchlorid, Chlorhexidin, Benzylalkohol, Glucose, Ethanol und quartäre Ammoniumsalze.

Von besonderem Vorteil ist die Zugabe viskositätserhöhender Mittel. Es hat sich nämlich überraschend herausgestellt, dass eine erhöhte Viskosität die Stabilisierung des Tränen-Films zusätzlich unterstützt und bessere Ergebnisse liefert als die Verwendung von Ectoin/Hydroxyectoin ohne viskositätserhöhenden Zusatz. Hintergrund ist vermutlich, dass das Ectoin/Hydroxyectoin länger auf dem Auge gehalten wird und daher über einen längeren Zeitraum zur Stabilisierung des Tränenfilms beitragen kann. Darüber hinaus wird die Einwirkung für den Patienten angenehmer.

Beispiele für viskositätserhöhende Mittel sind Celluloseether wie Hydroxyethylcellulose, Carboxymethylcellulose, Hydroxypropylmethylcellulose, Methylpropylcellulose, Methylcellulose, Methylethylcellulose, Ethylcellulose, Hydroxyethylmethylcellulose, Hydroxypropylcellulose, Ethylhydroxyethylcellulose. Andere Beispiele sind Polyethylenglycol, Polyvinylalkohole, Polyvinylpyrrolidon, Glykosaminoglykane, Proteoglykane, Cetylalkohol und Stearylalkohol bzw. Kombinationen hieraus (Cetylstearylalkohol), Polyacrylsäure, Polymethacrylsäure, Polyacrylamid, Polyether, Polyimine, Polyamide, Alginate, Xanthan, Polyuronide, Alginsäure, Carrageen, Chondroitinsulfat, Guaran, Hydroxypropylguaran und Stärkeacetat.

Die Konzentration der viskositätserhöhenden Mittel in der Zusammensetzung beträgt vorzugsweise 0,05 bis 10 Gew.-%, bevorzugt 0,1 bis 3 Gew.-%. Als zweckmäßig haben sich beispielsweise Konzentrationen für Celluloseether im Bereich von 0,2 bis 2,5 Gew.-%, für Polyethylenglykol im Bereich von 0,2 bis 1 Gew.-%, für Polyvinylalkohol von 0,1 bis 4 Gew.-% und für Polyacrylsäure von 0,1 bis 0,3 Gew.-% herausgestellt.

Befeuchtende oder feuchthaltende Stoffe sind z.B. Glycerin, Sorbitol, Trehalose, Betain, Dexpanthenol, 1,2-Propylenglycol, Xylit oder andere Polyalkohole.

Die Formulierungen der Erfindung können ebenfalls geeignete Puffersysteme oder andere Hilfsstoffe zur pH-Einstellung beeinhalten, um einen gewünschten pH-Wert einzustellen und aufrechtzuerhalten. Geeignete Puffersysteme sind Citrat, Phosphat, TRIS, Glycin, Borat, Acetat. Diese Puffersysteme können hergestellt werden aus Substanzen wie Zitronensäure, Mononatriumphosphat, Dinatriumphosphat, Glycin, Borsäure, Natriumtetraborat, Essigsäure oder Natriumacetat.

Die Zusammensetzungen können weitere Wirkstoffe enthalten, es ist jedoch insbesondere auch möglich und für die Behandlung oder Vorbeugung einer Augenerkrankung ausreichend, Zusammensetzungen zu verwenden, die lediglich Ectoin und/oder Hydroxyectoin bzw. entsprechende Salze, Ester oder Amide als Wirkstoff enthalten.

Weitere Wirkstoffe können beispielsweise andere kompatible Solute sein. Hier sind insbesondere Di-myo-Inositolphosphat (DIP), zyklisches 2,3-Diphosphoglycerat (cDPG), 1,1-Di-Glycerinphosphat (DGP), β-Mannosylglycerat (Firoin), β-Mannosylglyceramid (Firoin A), Di-Mannosyl-di-inositolphosphat (DMIP), Glucosylglycerin, Taurin, Betain, Citrullin, 4,5-Dihydro-2-methylimidazol-4-carbonsäure (DHMICA) und 4,5,6,7-Tetrahydro-2-methyl-1H-[1,3]-diazepin-4-S-carbonsäure (Homoectoin) sowie entsprechende Derivate, insbesondere Salze, Ester oder Amide zu nennen. Andere geeignete Wirkstoffe sind lokale Entzündungshemmer, z.B. Steroide, Cyclosporin A, Beta-Rezeptorenblocker.

Bestandteil der Zusammensetzung können auch Antibiotika sein. Hierzu gehören Gentamycin, Kanamycin, Neomycon, Tobramycin, Ciprofloxacin, Ofloxacin, Chlortetracyclin, Ciprofloxacin, Erythromycin, Fusidinsäure, Lomefloxacin, Levofloxacin und Oxytetracyclin.

Andere aktive Inhaltsstoffe natürlichen Ursprungs können sein: Omega-3-Fettsäuren, Augentrost, Digitalis, Euphrasia, Blaubeere, Kamille, Malve und Aloe vera.

Die Konzentration an Ectoin/Hydroxyectoin und/oder entsprechenden Salzen, Estern oder Amiden kann insbesondere in einem Bereich von 10 bis 500 mM, bevorzugt 50 bis 500 mM, besonders bevorzugt 100 bis 500 mM bzw. 100 bis 200 mM liegen. Diese Konzentrationen haben sich als geeignet zur Herbeiführung des erfindungsgemäßen Effekts erwiesen. Insbesondere kann der Anteil an Ectoin/Hydroxyectoin und/oder entsprechenden Salzen, Estern oder Amiden in der Zusammensetzung in einem Bereich von 0,1 bis 10 Gew.-% liegen. Eine gute Wirkung konnte beispielsweise in einem Bereich zwischen 0,5 und 2 Gew.-% beobachtet werden.

Um die Applikation und die Haltbarkeit der erfindungsgemäßen Zusammensetzung zu verbessern, wird die den Wirkstoff enthaltende Zusammensetzung in Form von Liposomen verabreicht. Dies ist insbesondere vorteilhaft, wenn die Zusammensetzung kein Konservierungsmittel enthält, was hinsichtlich des Einsatzzwecks im Auge bevorzugt ist. Entsprechende Verfahren zur Verkapselung sind grundsätzlich aus dem Stand der Technik bekannt. Die Verwendung von Liposomen ist auch deshalb besonders vorteilhaft, weil die die Membran des Liposoms ausbildenden Lipide, darunter Phospolipide und Fettsäuren, auch zur Bildung des Meibom-Films beitragen.

Des Weiteren betrifft die Erfindung auch eine Sprühvorrichtung, mit der die erfindungsgemäße Zusammensetzung auf das geöffnete oder geschlossene Auge appliziert werden kann. Die Sprühvorrichtung weist Mittel zur Zerstäubung der Zusammensetzung auf und verfügt sinnvollerweise über eine Austrittsöffnung, deren Größe an die Größe des Auges angepasst ist. Der Benutzer hält die Sprühvorrichtung in der Weise, dass die Austrittsöffnung vor dem Auge liegt, so dass bei Betätigung der Sprühvorrichtung das Auge mit der Zusammensetzung benetzt wird.

### Versuch 1

Meibom-Lipide wurden aus den Meibom-Drüsen von vier gesunden Freiwilligen gewonnen. Die Lipide wurden in Chloroform/Methanol (1:1, v/v) gelöst auf eine wässrige Subphase (PBS, phosphatgepufferte Salzlösung, pH 7,4, T = 20°C) aufgebracht. Nach einer Äquilibrierungszeit von 10 - 15 min wurden die Monolayer mit 2,9 cm²/min komprimiert. Die Subphase wurde mit unterschiedlichen Ectoin-Konzentrationen versehen.

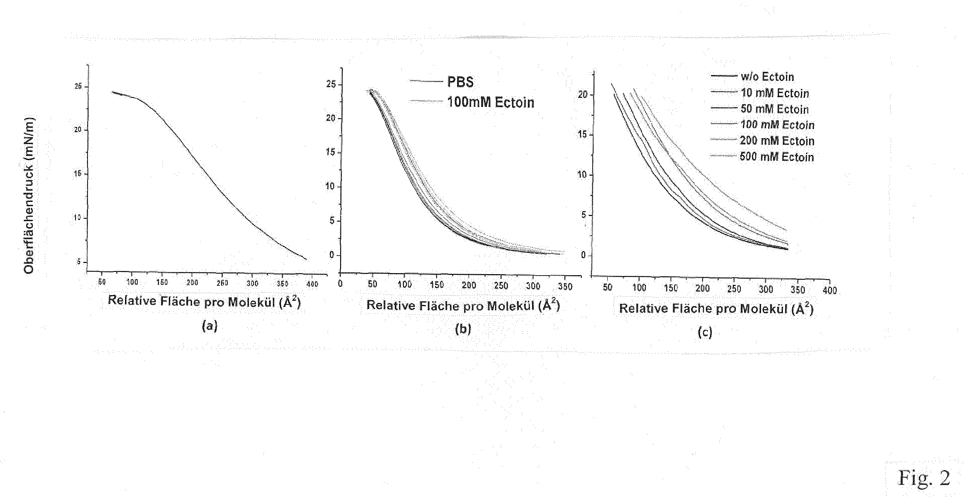

Das Ergebnis der Kompression des Lipidfilms auf einer PBS-Subphase ohne Ectoin ist in Figur 2 a dargestellt. Man beobachtet keine Phasenübergänge bis zu einem Oberflächendruck von 20 mN/m. In Gegenwart von Ectoin verschiebt sich die Isotherme in Richtung größerer Fläche pro Molekül, verbunden mit einer größeren Fläche, die durch die Lipidkopfgruppen eingenommen wird. Ectoin vergrößert somit die Zwischenräume zwischen den Molekülen. Der Effekt ist, wie sich Figur 2 c entnehmen lässt, konzentrationsabhängig. In Figur 2 b sind Kompressions-Expansionskurven gezeigt; hinsichtlich der Hysterese werden keine signifikanten Unterschiede beobachtet.

Die Versuche zeigen, dass die einzelnen Moleküle aus der Lipidschicht in Gegenwart von Ectoin eine größere Fläche einnehmen, d. h. die Lipidschicht insgesamt fluider und weniger starr wird und somit weniger zum Reißen neigt.

### Versuch 2

Die Topographie der Oberfläche der Lipidschichten wurde mit Hilfe eines Rasterkraftmikroskops (atomic force microscopy, AFM) untersucht. Hierzu wurde die Lipidschicht mittels Langmuir-Blodgett-Technik auf eine feste Oberfläche übertragen, anschließend wurden die AFM-Messungen bei Drücken von 5 mN/m, 20 mN/m und 23,5 mN/m und 20°C durchgeführt. Bei einem relativ niedrigen Druck von 5 mN/m konnten kleine faserartige Strukturen von bis zu ca. 5 nm Höhe über die gesamte Fläche verteilt beobachtet werden. Hierbei könnte es sich um Aufschichtungen von hydrophoben Lipiden wie Cholesterinestern und Triacylglyceriden handeln. Bei höheren Drücken von 20 mN/m nahm die Zahl der faserartigen Strukturen zu.

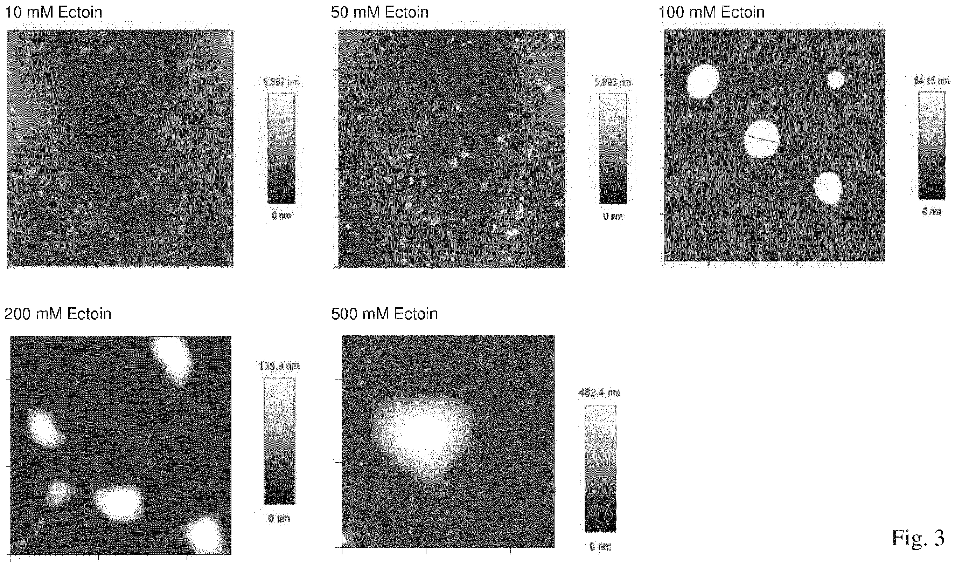

In Gegenwart von 100 mM Ectoin wurden tröpfchenartige Strukturen beobachtet, die eine Höhe von bis zu ca. 300 nm aufweisen. Bei höheren Ectoinkonzentrationen nahm der Durchmesser der tröpfchenartigen Strukturen zu. Auch in Gegenwart von 50 mM Ectoin kann die Bildung solcher Strukturen beobachtet werden, allerdings noch vergleichsweise verstreut und deutlich kleiner. Es konnte gezeigt werden, dass die tröpfchenartigen Strukturen fluider sind als die übrigen Bereiche der Lipidschicht, was für eine Konzentration von Triacylglyeriden spricht, die weniger stark molekular organisiert sind als Phospholipide und Cholesterinester. Das Ergebnis wird in Figur 3 wiedergegeben.

Insgesamt zeigen somit auch die AFM-Untersuchungen, dass die Lipidschicht in Gegenwart von Ectoin fluider wird, was mit einer Verringerung der Gefahr des Reißens der Lipidschicht verbunden ist.

Ähnliche Ergebnisse konnten mit künstlichen Meibom-Filmen erzielt werden. Diese wurden erzeugt, indem ein Phospholipid (DPPC, 1,2-Dipalmitoyl-sn-glycero-3-phosphocholin), ein Cholesterinester (CP, Cholesteryl-3-palmitat) und ein Triacylglycerid (DPOG, 1,3-Dipalmitoyl-2-oleoylglycerol) in unterschiedlichen Verhältnissen miteinander kombiniert wurden.

### Versuch 3

In einer Studie wurden 64 Patienten untersucht, die an einer leichten bis mittleren Form der Keratoconjunctivitis sicca leiden. Sämtliche Patienten waren älter als 18 Jahre und wiesen akute Symptome auf (Schweregrad 1 - 3 gemäß Dry Eye Work Shop (DEWS); Research in dry eye: report oft the Research Subcommittee of the International Drye Eye WorkShop. Ocul Surf 2007; 5(2): 179 - 193). Die Mehrheit (72 %) der Patienten war weiblich. Für jeweils mindestens ein Auge betrug die Tear break-up time (TBUT), d. h. die Zeit, bis nach dem Blinzeln wieder trockene Stellen auf der Cornea auftreten, weniger als 10 sec. Die TBUT wurde als Hauptparameter für die Bewertung der Behandlung verwendet.

34 Patienten wurden über einen Zeitraum von 28 Tagen mit einer Ectoin enthaltenden Zusammensetzung, die übrigen 30 Patienten mit Hyaluronsäure als Referenz behandelt. Von den Patienten beendeten 59 die Studie wie vorgesehen; 5 Patienten schieden vorher aus.

Im Durchschnitt verbesserte sich die TBUT innerhalb des 28tägigen Beobachtungszeitraums bei mit Ectoin behandelten Patienten um 2,6 sec (p=0,0011) und bei den mit Hyaluronsäure behandelten Patienten um 1,1 sec (p=0,1686). Insbesondere die Patienten mit starker Keratoconjunctivitis sicca und einer TBUT < 5 sec zeigten bei Behandlung mit Ectoin eine stärkere Verbesserung als bei Behandlung mit Hyaluronsäure.

Die Lebensqualität der Patienten wurde anhand des OSDI (ocular surface disease index) bestimmt. Die OSDI-Bewertung besteht aus 12 Fragen, die von den Patienten beantwortet werden auf einer Skala von 0 bis 4 (0 = nie; 4 = immer). Desto höher der OSDI-Wert, desto stärker wird das tägliche Leben von der Erkrankung beeinträchtigt. Bei den mit Ectoin behandelten Patienten konnte im Untersuchungszeitraum eine Abnahme von 17,7, bei den mit Hyaluronsäure behandelten Patienten von 17,2 Punkten beobachtet werden.

Gemäß dem Schirmer II-Test wurde der Tränenfluss untersucht. Dieser war sowohl zu Beginn (8,8 mm/5 min gegenüber 16,0 mm/5 min) als auch am Ende (10,6 mm/5 min gegenüber 16,8 mm/5 min) der Behandlung für die Ectoin-Patienten niedriger als für die Hyaluronsäure-Patienten, wobei für die mit Ectoin behandelten Patienten eine deutlichere Verbesserung beobachtet werden konnte als für die mit Hyaluronsäure behandelten Patienten (1,8 mm/5 min gegenüber 0,8 mm/5 min).

Die Einschätzung der Effektivität der Behandlung war zwischen den Patientengruppen vergleichbar, nach Einschätzung der Patienten jedoch für Ectoin etwas besser als für Hyaluronsäure (0,9 gegenüber 0,6 Punkten bei der Bewertung). Insbesondere das Fremdkörpergefühl und das Gefühl müder Augen wurden nach der Behandlung als verbessert bewertet. 78,8 % der mit Ectoin und 71,4 % der mit Hyaluronsäure behandelten Patienten würden die Verwendung fortsetzen. Sowohl die Behandlung mit Ectoin als auch die Referenzbehandlung wurden allgemein gut vertragen.

## Patentansprüche

1. Zusammensetzung, enthaltend als Wirkstoff Ectoin, Hydroxyectoin und/oder Salze, Ester oder Amide dieser Verbindungen zur Verwendung in einem Verfahren zur Behandlung und/oder Prophylaxe von Erkrankungen des Auges, die mit einer Störung des Tränenfilms in Zusammenhang stehen, wobei die Erkrankung eine hyperevaporative Keratoconjunctivitis sicca ist, die auf eine Fehlfunktion der Meibom-Drüsen zurückzuführen ist, und der Wirkstoff eine Fluidisierung der durch die Meibom-Drüsen erzeugten Lipidschicht bewirkt und wobei die Zusammensetzung in Form von Liposomen vorliegt.

2. Zusammensetzung zur Verwendung nach Anspruch 1, **dadurch gekennzeichnet, dass** die Konzentration an Ectoin, Hydroxyectoin und/oder Salzen, Estern oder Amiden dieser Verbindungen in der Zusammensetzung 10 bis 500 mM beträgt.

3. Zusammensetzung zur Verwendung nach Anspruch 2, **dadurch gekennzeichnet, dass** die Konzentration an Ectoin, Hydroxyectoin und/oder Salzen, Estern oder Amiden dieser Verbindungen in der Zusammensetzung 100 bis 500 mM beträgt.

4. Zusammensetzung zur Verwendung nach einem der Ansprüche 1 bis 3, **dadurch gekennzeichnet, dass** die Zusammensetzung ein oder mehrere viskositätserhöhende Mittel enthält.

5. Zusammensetzung zur Verwendung nach Anspruch 4, **dadurch gekennzeichnet, dass** die viskositätserhöhenden Mittel ausgewählt sind aus: Celluloseether, Polyethylenglycol, Polyvinylalkohol, Polyvinylpyrrolidon, Glykosaminoglykane, Proteoglykane, Cetylalkohol und Stearylalkohol bzw. Kombinationen hieraus (Cetylstearylalkohol), Polyacrylsäure, Polymethacrylsäure, Polyacrylamid, Polyether, Polyimine, Polyamide, Alginate, Xanthan, Polyuronide, Alginsäure, Carrageen, Chondroitinsulfat, Guaran, Hydroxypropylguaran und Stärkeacetat.

6. Zusammensetzung zur Verwendung nach Anspruch 5, **dadurch gekennzeichnet, dass** der Celluloseether ausgewählt ist aus: Hydroxyethylcellulose, Carboxymethylcellulose, Hydroxypropylmethylcellulose, Methylpropylcellulose, Methylcellulose, Methylethylcellulose, Ethylcellulose, Hydroxyethylmethylcellulose, Hydroxypropylcellulose, Ethylhydroxyethylcellulose.

7. Zusammensetzung nach einem der Ansprüche 4 bis 6, **dadurch gekennzeichnet, dass** die Konzentration der viskositätserhöhenden Mittel in der Zusammensetzung 0,05 bis 10 Gew.-%, bevorzugt 0,1 bis 3 Gew.-% beträgt.

8. Zusammensetzung zur Verwendung nach einem der Ansprüche 1 bis 7, **dadurch gekennzeichnet, dass** die Zusammensetzung eine wässrige Lösung ist.

9. Zusammensetzung zur Verwendung nach einem der Ansprüche 1 bis 8, **dadurch gekennzeichnet, dass** es sich um eine isotonische, hypotonische oder hypertonische Zusammensetzung handelt.

10. Zusammensetzung zur Verwendung nach einem der Ansprüche 1 bis 9, **dadurch gekennzeichnet, dass** die Zusammensetzung in Form von Augentropfen vorliegt.

11. Zusammensetzung zur Verwendung nach einem der Ansprüche 1 bis 7, **dadurch gekennzeichnet, dass** die Zusammensetzung in Form einer Creme oder eines Gels vorliegt.

12. Sprühvorrichtung, enthaltend eine Zusammensetzung zur Verwendung nach einem der Ansprüche 1 bis 10, zur Applikation der Zusammensetzung auf ein geöffnetes oder geschlossenes Auge.

## Claims

1. Composition, containing as active ingredient ectoine, hydroxyectoine and/or salts, esters or amides of said compounds for use in a method for treatment and/or prophylaxis of diseases of the eyes associated with a disorder of the tear film, the disease being a hyperevaporative keratoconjunctivitis sicca based on a malfunction of the Meibom glands and the active ingredient effecting a fluidisation of the lipid layer formed by the Meibom glands, and the composition being present in the form of liposomes.

2. Composition for use according to claim 1, **characterised in that** the concentration of ectoine, hydroxyectoine and/or salts, esters or amides of said compounds in the composition amounts to 10 to 500 mM.

3. Composition for use according to claim 2, **characterised in that** the concentration of ectoine, hydroxyectoine and/or salts, esters or amides of said compounds in the composition amounts to 100 to 500 mM.

4. Composition for use according to any one of claims 1 to 3, **characterised in that** the composition contains one or more viscosity enhancing agents.

5. Composition for use according to claim 4, **characterised in that** viscosity enhancing agents are selected from: cellulose ether, polyethylene glycol, polyvinyl alcohol, polyvinyl pyrrolidone, glycosaminoglycans, proteoglycans, cetyl alcohol and stearyl alcohol or combinations thereof (cetyl stearyl alcohol), respectively, polyacrylic acid, polymethacrylic acid, polyacrylamide, polyethers, polyimines, polyamides, alginates, xanthan gum, polyuronides, alginic acid, carrageenan, chondroitin sulphate, guar gum, hydroxypropyl guar gum, and starch acetate.

6. Composition for use according to claim 5, **characterised in that** the cellulose ether is selected from: hydroxyethyl cellulose, carboxymethyl cellulose, hydroxypropyl methyl cellulose, methyl propyl cellulose, methyl cellulose, methyl ethyl cellulose, ethyl cellulose, hydroxyethyl methyl cellulose, hydroxypropyl cellulose, ethyl hydroxyethyl cellulose.

7. Composition according to any one of claims 4 to 6, **characterised in that** the concentration of the viscosity enhancing agents in the composition amount to 0.05 to 10 % by weight, preferably 0.1 to 3 % by weight.

8. Composition for use according to any one of claims 1 to 7, **characterised in that** the composition is an aqueous solution.

9. Composition for use according to any one of claims 1 to 8, **characterised in that** it is an isotonic, hypotonic or hypertonic composition.

10. Composition for use according to any one of claims 1 to 9, **characterised in that** the composition is in the form of eye drops.

11. Composition for use according to any one of claims 1 to 7, **characterised in that** the composition is in the form of a cream or of a gel.

12. Spray device, containing a composition for use according to any one of claim 1 to 10, for applying the composition to an open or closed eye.

## Revendications

1. Composition, contenant en tant qu'un principe actif de l'ectoine, de l'hydroxyectoine et/ou des sels, esters ou amides desdits composés pour utilisation dans une méthode de traitement et/ou de prophylaxie d'affections des yeux associées avec un trouble du film de larmes, l'affection étant une kératoconjonctivite sicca hyperévaporative basée sur un dysfonctionnement des glandes de Meibom et le principe actif effectuant une fluidisation de la couche lipidique formée par les glandes de Meibom, et la composition étant présente sous forme des liposomes.

2. Composition pour utilisation selon la revendication 1, **caractérisée en ce que** la concentration de l'ectoine, l'hydroxyectoine et/ou des sels, esters ou amides desdits composés dans la composition se monte à 10 à 500 mM.

3. Composition pour utilisation selon la revendication 2, **caractérisée en ce que** la concentration de l'ectoine, l'hydroxyectoine et/ou des sels, esters ou amides desdits composés dans la composition se monte à 100 à 500 mM.

4. Composition pour utilisation selon l'une des revendications 1 à 3, **caractérisée en ce que** la composition contient un ou plusieurs agents accroissant la viscosité.

5. Composition pour utilisation selon la revendication 4, **caractérisée en ce que** les agent accroissant la viscosité sont sélectionnés : de l'éther cellulosique, du polyéthylèneglycol, de l'alcool polyvinylique, de la polyvinylpyrrolidone, des glycosaminoglycanes, des protéoglycanes, de l'alcool de cétylique et de l'alcool stéarique ou des combinations de ceux-ci (de l'alcool stéarique cétylique), respectivement, de l'acide polyacrylique, de l'acide polyméthacrylique, du polyacrylamide, des polyéthers, des polyimines, des polyamides, des alginates, de la gomme de xanthane, des polyuronides, de l'acide alginique, du carraghénane, de la chondroïtine sulfate, de la gomme de guar, de la gomme de guar hydroxypropylène, et de l'acétate d'amidon.

6. Composition pour utilisation selon la revendication 5, **caractérisée en ce que** l'éther cellulosique est sélectionné : de l'hydroxyéthylcellulose, de la carboxyméthylcellulose, de l'hydroxypropylméthylcellulose, de la méthylpropylcellulose, de la méthylcellulose, de la méthyléthylcellulose, de l'ethylcellulose, de l'hydroxyéthylméthylcellulose, de l'hydroxypropylcellulose, de l'éthylhydroxyéthylcellulose.

7. Composition selon l'une des revendications 4 à 6, **caractérisée en ce que** la concentration des agents accroissant la viscosité dans la composition se monte à 0.05 à 10 % en poids, de préférence 0.1 à 3 % en poids.

8. Composition pour utilisation selon l'une des revendications 1 à 7, **caractérisée en ce que** la composition s'agit d'une solution aqueuse.

9. Composition pour utilisation selon l'une des revendications 1 à 8, **caractérisée en ce qu'**il est une composition isotonique, hypotonique ou hypertonique.

10. Composition pour utilisation selon l'une des revendications 1 à 9, **caractérisée en ce que** la composition est sous forme des gouttes ophtalmiques.

11. Composition pour utilisation selon l'une des revendications 1 à 7, **caractérisée en ce que** la composition est sous forme d'une crème ou d'un gel.

12. Dispositif de pulvérisation, contenant une composition pour utilisation selon l'une des revendications 1 à 10, pour appliquer la composition à un œil ouvert ou fermé.
